# FASCICULE DE BREVET EUROPEEN

(11) **EP 1 278 532 B1**
(45) Date de publication et mention de la délivrance du brevet: **16.01.2008**
(21) Numéro de dépôt: 01929748.0
(22) Date de dépôt: 27.04.2001
(51) Int. Cl.: A61K 36/63

(54) **EXTRAIT DE VEGETAL DE L'ESPECE OLEA EUROPAEA COMME INHIBITEUR DE NO-SYNTHASE ET UTILISATIONS**
EXTRAKT AUS EINER PFLANZE DER SPEZIES OLEA EUROPAEA ALS INHIBITOR DER NO-SYNTHASE UND VERWENDUNGEN
PLANT EXTRACT OF THE OLEA EUROPAEA SPECIES AS NO-SYNTHASE INHIBITOR AND USES

(30) Priorité: 28.04.2000 FR 0005522
(43) Date de publication de la demande: 29.01.2003
(73) Titulaire: L'ORÉAL, 75008 Paris (FR)
(72) Inventeur: CALS-GRIERSON, Marie-Madeleine, F-92190 Meudon (FR); PELLETIER, Pascale, F-92160 Antony (FR)
(74) Mandataire: Allab, Myriam
(86) Numéro de dépôt international: PCT/FR2001/001315
(87) Numéro de publication internationale: WO 2001/082940

(56) Documents cités:
- EP-A- 0 937 455
- BENAVENTE-GARCIA O ET AL: "ANTIOXIDANT ACTIVITY OF PHENOLICS EXTRACTED FROM OLEA EUROPAEA L. LEAVES" FOOD CHEMISTRY, ELSEVIER SCIENCE PUBLISHERS LTD, GB, vol. 68, no. 4, mars 2000 (2000-03), pages 457-462, XP000987105 ISSN: 0308-8146
- TUTOUR B LE ET AL: "ANTIOXIDATIVE ACTIVITIES OF OLEA EUROPAEA LEAVES AND RELATED PHENOLIC COMPOUNDS" PHYTOCHEMISTRY,GB,PERGAMON PRESS, vol. 31, no. 4, 1992, pages 1173-1178, XP000565805 ISSN: 0031-9422

## Description

La présente invention a pour objet l'utilisation d'une quantité efficace d'au moins un extrait d'au moins un végétal de l'espèce *Olea europaea*, dans un milieu physiologiquement acceptable, dans une composition ou pour la préparation d'une composition, l'extrait ou la composition étant destinés à inhiber la NO-synthase.

Le terme NO-synthase recouvre une famille d'enzymes qui assurent la catalyse enzymatique de la L-arginine en citrulline, catalyse au cours de laquelle est produit un médiateur gazeux aux multiples fonctions, le monoxyde d'azote ou NO.

Les NO-synthases existent sous trois formes, deux formes constitutives, nomenclature regroupant la NO-synthase neuronale (ou NOS 1) et la NO-synthase endothéliale (ou NOS 3), et la forme inductible (ou NOS 2) (Medecine/Sciences, 1992, 8, pp. 843-845).
On comprend par ailleurs dans le texte que sans indication contraire le terme NO-synthase recouvre l'ensemble des isoformes de l'enzyme.

Ainsi, selon l'invention on entend par inhibiteurs de NO-synthase, tout produit qui in fineconduit, nonobstant l'isoforme de NO-synthase, à la diminution de la concentration de NO. On peut citer à titre d'exemple les produits qui diminuent la quantité de NO-synthase active, qui bloquent l'activité enzymatique de la NO-synthase ou son induction ou qui inhibent l'activité du NO produit.

Le monoxyde d'azote possède de par sa structure un électron supplémentaire le rendant extrêmement réactif chimiquement. Il est notoire que de tels composés sont nocifs et l'on cherche à limiter au mieux leur production. C'est ainsi que dans le cas du monoxyde d'azote les inhibiteurs de NO-synthase ont été largement étudiés.
Le NO est une molécule signal multifonctionnelle active dans une grande variété de systèmes et de tissus du corps. Outre ses effets dommageables pour les cellules liés à son hyperréactivité due à sa structure comprenant un électron supplémentaire, elle est reconnue entre autre comme intervenant particulièrement dans le système cardiovasculaire (régulateur de la pression sanguine avec effet vasodilatateur, inhibiteur de l'agrégation plaquettaire avec effet anticoagulant), dans le système nerveux (mémoire, modulation de la libération des neurotransmetteurs), dans le système immunologique (modulation des défenses immunitaires, inflammation, implication dans les pathologies auto-immunes).

Il est maintenant bien admis que le NO joue un rôle prépondérant dans la peau. Le NO peut être synthétisé par toutes les variétés de cellules constituant la peau et à ce titre il intervient dans de multiples et complexes processus de régulation tels que la régulation de la différenciation et/ou de la prolifération cellulaire, de la vasodilatation, de la mélanogenèse, de la réponse aux variations environnementales (homéostasie).
Son implication dans la différenciation et la prolifération cellulaire (effet stimulateur), particulièrement des kératinocytes, l'associe aussi bien à la croissance de l'épiderme et à la cicatrisation qu'aux désordres hyperprolifératifs (psoriasis).
Du fait de son hyperréactivité électronique pouvant entraîner une dégradation voire une destruction des cellules, le NO est impliqué dans les processus apoptotiques et dans le vieillissement intrinsèque et/ou extrinsèque de la peau.
Il intervient dans les processus immunologiques et inflammatoires cutanés. Il est en effet communément admis que le NO joue un rôle dans les réactions d'hypersensibilité de contact, dans les manifestations allergiques cutanées, dans la réponse immunitaire de la peau. De même, outre son rôle proinflammatoire direct, il est le médiateur entre les neuropeptides comme la substance P et/ou le peptide associé au gène de la calcitonine (calcitonin gene related peptide ou CGRP) dans les processus inflammatoires neurogéniques cutanés, d'ou son implication dans les phénomènes de peau dite sensible.
L'implication du NO dans la vasodilatation fait qu'il est associé aux érythèmes cutanés, particulièrement les érythèmes induits par les rayonnements ultra-violets, aux éruptions érythémateuses localisées ou diffuses de la peau comme celles causées par les drogues les toxines et/ou les infections virales ou bactériennes, à la rosacée.
Le NO est reconnu comme intermédiaire dans la mélanogenèse induite par les rayonnements ultra-violets de type B (UVB). Il serait aussi un des facteurs intervenant dans les désordres de type hypermélanose.
Le NO semble également impliqué dans le contrôle de la sudation ainsi que dans celui de la lipolyse (effet inhibiteur) ou encore dans la chute des cheveux.
Enfin, le NO est connu pour avoir une influence sur la fonction barrière de la peau et donc sur l'hydratation de celle-ci (effet inhibiteur).

On comprend donc l'intérêt qui existe à disposer d'inhibiteurs des NO-synthases. A cet égard de nombreux inhibiteurs ont déjà été proposés dans l'art antérieur. On peut citer plus particulièrement la N^{G}-monométhyl-L-arginine (NMMA), l'ester méthylé de la N^{G}-nitro-L-arginine (NAME), la N^{G}-nitro-L-arginine (NNA), la N^{G}-amino-L-arginine (NAA), la N^{G}.N^{G}-diméthyl-arginine (la diméthylarginine asymétrique, dénommée ADMA), le chlorure de diphénylèneiodonium, le 2-(4-carboxyphényl)-4,4,5,5-tetraméthylimidazoline-1-oxy-3-oxyde, la 7-nitroindazole, la N(5)-(1-iminoéthyl)-L-ornithine, l'aminoguanidine, la canavanine et l'ebselen.
Sans mettre en doute l'efficacité de ces produits, on note qu'il s'agit de composés chimiques qui peuvent induire des désagréments chez l'utilisateur voire des effets secondaires néfastes, qui de manière générale préfère utiliser des produits naturels.
Le but de la présente invention est de fournir un nouvel inhibiteur de NO-synthase qui plus est un inhibiteur naturel de NO-synthase.

De manière surprenante et inattendue, la demanderesse a démontré qu'un extrait d'au moins un végétal de l'espèce *Olea europaea* présente la propriété d'être un inhibiteur de NO-synthase, particulièrement de la NO-synthase inductible (NOS 2) ce qui en fait un bon candidat pour des utilisations dans des applications où il s'avère intéressant d'utiliser un inhibiteur de NO-synthase, particulièrement en cosmétique.

Les extraits de végétal de l'espèce *Olea europaea* sont connus dans l'art antérieur comme favorisant la vasodilatation ou encore comme pouvant être utilisés dans de compositions destinées aux soins de la peau et/ou des cheveux.

Mais à la connaissance de la demanderesse il n'a jamais été décrit qu'un extrait d'au moins un végétal de l'espèce *Olea europaea* présente la propriété d'être un inhibiteur de NO-synthase, particulièrement de la NO-synthase inductible (NOS 2).

L'invention a donc pour objet premier l'utilisation d'une quantité efficace d'au moins un extrait d'au moins un végétal de l'espèce *Olea europaea*, dans un milieu physiologiquement acceptable, dans une composition ou pour la préparation d'une composition, l'extrait ou la composition étant destinés à inhiber la NO-synthase, particulièrement de la NO-synthase inductible (NOS 2).

DE 3901288 décrit des compositions destinées à limiter la chute des cheveux contenant notamment un extrait de feuilles d'olivier, en mélange avec nicotinamide, D-alpha tocophérol, D-pantothenol et lécithine.
Pieroni et al (Pharmazie, 51, 765-768, 1996) rapporte un effet anti-inflammatoire de décoction préparées à partir des parties vertes de l'espèce Olea,
EP 937435 enseigne que des extrait de feuille d'Olea europea peuvent être utilisés comme piégeurs de radicaux libres.

Par milieu physiologiquement acceptable, on comprend un milieu compatible avec la peau, les muqueuses, les ongles, les cheveux.

L'invention a pour second objet l'utilisation d'une quantité efficace d'au moins un extrait d'au moins un végétal de l'espèce *Olea europaea,* dans un milieu physiologiquement acceptable, dans une composition ou pour la préparation d'une composition, l'extrait ou la composition étant destinés à une application dans tous les domaines dans lesquels une inhibition des NO-synthases, particulièrement de la NO-synthase inductible (NOS 2), s'avère nécessaire, particulièrement dans le domaine cutané et/ou capillaire.

Ainsi, l'extrait de végétal de l'espèce *Olea europaea* ou la composition le contenant peuvent être utilisés pour ralentir voire inhiber la différenciation et/ou la prolifération cellulaire, et/ou la vasodilatation, et/ou la mélanogenèse, et/ou la réponse aux variations environnementales (homéostasie).

Ainsi, l'invention a pour troisième objet l'utilisation d'une quantité efficace d'au moins un extrait d'au moins un végétal de l'espèce *Olea europaea*, dans un milieu physiologiquement acceptable, dans une composition ou pour la préparation d'une composition, l'extrait ou la composition étant destinés à ralentir voire inhiber la différenciation et/ou la prolifération cellulaire, particulièrement à réguler la croissance de l'épiderme et/ou à traiter les désordres hyperprolifératifs comme par exemple le psoriasis.

L'invention a pour quatrième objet l'utilisation d'une quantité efficace d'au moins un extrait d'au moins un végétal de l'espèce *Olea europaea*, dans un milieu physiologiquement acceptable, dans une composition ou pour la préparation d'une composition, l'extrait ou la composition étant destinés à inhiber la dégradation et/ou la destruction des cellules, particulièrement des cellules de la peau, très particulièrement des kératinocytes et/ou à traiter le vieillissement intrinsèque et/ou extrinsèque des cellules, particulièrement des cellules de la peau.

L'invention a pour cinquième objet l'utilisation d'une quantité efficace d'au moins un extrait d'au moins un végétal de l'espèce *Olea europaea,* dans un milieu physiologiquement acceptable, dans une composition ou pour la préparation d'une composition, l'extrait ou la composition étant destinés

à diminuer voire inhiber les processus inflammatoires neurogéniques cutanés, et donc à traiter les peaux dites sensibles.

L'invention a pour sixième objet l'utilisation d'une quantité efficace d'au moins un extrait d'au moins un végétal de l'espèce *Olea europaea,* dans un milieu physiologiquement acceptable, dans une composition ou pour la préparation d'une composition, l'extrait ou la composition étant destinés à traiter la rosacée et/ou les érythèmes cutanés, particulièrement les érythèmes induits par les rayonnements ultra-violets et/ou les éruptions érythémateuses localisées ou diffuses de la peau comme celles causées par les drogues les toxines et/ou les infections virales ou bactériennes.

L'invention a pour septième objet l'utilisation d'une quantité efficace d'au moins un extrait d'au moins un végétal de l'espèce *Olea europaea*, dans un milieu physiologiquement acceptable, dans une composition ou pour la préparation d'une composition, l'extrait ou la composition étant destinés à inhiber la mélanogenèse induite par les rayonnements ultra-violets de type A et/ou B et/ou à traiter les désordres de type hypermélanose.

L'invention a pour huitième objet l'utilisation d'une quantité efficace d'au moins un extrait d'au moins un végétal de l'espèce *Olea europaea*, dans un milieu physiologiquement acceptable, dans une composition ou pour la préparation d'une composition, extrait ou la composition étant destinés à contrôler la sudation et/ou à stimuler la lipolyse et/ou à renforcer la fonction barrière de la peau et/ou à stimuler l'hydratation de la peau.

Selon l'invention, la composition comprenant l'extrait peut être une composition cosmétique ou dermatologique. Préférentiellement selon l'invention, la composition est une composition cosmétique.

Préférentiellement selon l'invention, l'extrait ou la composition le comprenant est appliqué sur la peau de manière topique.

Le végétal de l'espèce *Olea europaea* est l'une des 35 espèces végétales appartenant au genre Olea qui lui même appartient à la famille des Oléacées.

Bien entendu, l'extrait peut être préparé à partir d'au moins l'une quelconque des nombreuses variétés associées à chacune des espèces végétales appartenant au genre Olea. On comprend donc que dans le texte le terme *Olea europaea* doit s'entendre comme désignant l'une quelconque des nombreuses variétés végétales associées à chacune des espèces végétales appartenant au genre Olea et particulièrement comme désignant l'espèce *Olea europaea*.

L'extrait de végétal de l'espèce *Olea europaea* utilisé selon l'invention obtenu à partir de matériel végétal issu de parties de plante comme les feuilles.

Par cellules végétales dédifférenciées, on entend toute cellule végétale ne présentant aucun des caractères d'une spécialisation particulière et capable de vivre par elle-même et non en dépendance avec d'autres cellules.

Préférentiellement selon l'invention on utilise un extrait préparé à partir de feuilles vertes.

L'extrait d'au moins un végétal du genre Olea peut être tout extrait préparé à partir de tout matériel végétal issu d'au moins un végétal du genre Olea cultivé *in vivo* ou issu de culture *in vitro.*

Par culture *in vivo* on entend toute culture de type classique c'est à dire en sol à l'air libre ou en serre, ou encore hors sol.

Par culture *in vitro,* on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Préférentiellement selon l'invention on utilise un végétal issu de culture *in vivo.*

Toute méthode d'extraction connue de l'homme du métier peut être utilisée pour préparer l'extrait contenu dans la composition selon l'invention.

On peut en particulier citer les extraits aqueux, alcooliques ou utilisant un solvant organique.
Par solvant aqueux on entend tout solvant constitué totalement ou pour part d'eau. On peut citer ainsi l'eau elle-même, les solvants hydroalcooliques en toute proportion ou encore les solvants constitués d'eau et d'un composé comme le propylène glycol en toute proportion.

Parmi les solvants alcooliques on peut citer notamment l'éthanol.

On peut également utiliser un extrait préparé par la méthode décrite dans la demande de brevet français n° 95-02379 déposée par la demanderesse.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.
D'autre part, la première étape peut avantageusement être remplacée par une opération de congélation simple des tissus végétaux (par exemple à -20°C), suivie d'une extraction aqueuse reprenant les deuxièmes et troisième étapes ci-dessus décrites.

Quel que soit le mode de préparation utilisé selon l'invention des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélanger dans un solvant approprié avant utilisation.

On peut encore citer des extraits commerciaux tels qu'un extrait sec de végétal de l'espèce *Olea europaea* vendu par la société Vinyals sous le nom commercial extrait sec d'olivier ou encore un extrait aqueux sec de feuille d'olivier vendu par la société Biologia & Technologia sous le nom commercial Eurol BT.

Préférentiellement selon l'invention on utilise un extrait sec de végétal de l'espèce *Olea europaea* vendu par la société Vinyals sous le nom commercial extrait sec d'olivier.

Selon l'invention, la quantité d'au moins un extrait d'au moins un végétal de l'espèce *Olea europaea* utilisée dans la composition est bien entendu fonction de l'effet recherché et peut donc varier dans une large mesure.

Pour donner un ordre de grandeur, selon l'invention, l'extrait peut être utilisé en une quantité représentant de 10⁻⁴% à 20% du poids total de la composition et préférentiellement en une quantité représentant de 5.10⁻³% à 10% du poids total de la composition.

Bien entendu, selon l'invention l'extrait d'au moins un végétal de l'espèce *Olea europaea* peut être associé à d'autres inhibiteurs de NO-synthases comme par exemple le lipochroman-6, ou d'autres extraits végétaux comme par exemple un extrait de *Ginkgo biloba*, un extrait de *Vitis vinifera* ou encore un extrait de thé vert ou de cacao.

L'invention a pour neuvième objet un procédé de traitement cosmétique en vue de traiter les désordres liés à la synthèse du NO, caractérisé par le fait que l'on utilise par application sur la peau, sur les cheveux, et/ou sur les muqueuses, une composition cosmétique comprenant au moins un extrait d'au moins un végétal de l'espèce *Olea europaea* dans un milieu physiologiquement acceptable.

Le procédé de traitement cosmétique de l'invention vise à améliorer l'aspect de l'individu atteint par les désordres dus à la synthèse du NO.

Le procédé de traitement cosmétique de l'invention peut être mis en oeuvre notamment en appliquant les compositions cosmétiques telles que définies ci-dessus, selon la technique d'utilisation habituelle de ces compositions. Ainsi par exemple il est possible d'effectuer des applications de crèmes, de gels, de sérums, de lotions, de laits de démaquillage ou de compositions anti-solaires sur la peau ou sur les cheveux secs, des applications d'une lotion pour cheveux sur cheveux mouillés, de shampooings, ou encore des applications de dentifrice sur les gencives.

Quelque soit la forme de la composition selon l'invention dans laquelle l'extrait est utilisé, celle-ci peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosacée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.
Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un extrait d'au moins un végétal de l'espèce *Olea europaea* à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox
- les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits végétaux ou d'origine microbienne,
- les peptides et leur dérivés comme par exemple le tripeptide Lys-Pro-Val.

Les exemples et compositions suivants illustrent l'invention sans la limiter aucunement. Dans les compositions les proportions indiquées sont des pourcentages en poids.

### Exemple 1 : Activité biologique d'extraits commerciaux de végétal de l'espèce Olea europaea :

Le test est réalisé avec deux extraits commerciaux, à savoir :
1 : extrait aqueux sec de végétal de l'espèce *Olea europaea* vendu par la société Vinyals sous le nom commercial d'extrait sec d'olivier.
2 : extrait aqueux sec de feuille d'olivier vendu par la société Biologia & Technologia sous le nom commercial Eurol BT.

L'activité de l'extrait sur la NO-synthase inductible a été évalué dans le test décrit par Heck et col. (J.B.C., Vol. 267, N°30, 21277-21280, 25 octobre 1992). Ce test a pour objectif de montrer la diminution de la concentration en nitrate et nitrite, in fine, après stimulation de la NO-synthase 2.

Les contrôles suivants ont été introduits dans le tests :
A : contrôle positif (induction de l'enzyme) : mélanges d'interféron-y (1000 U/ml) et d'Interleukine 1-β (100 U/ml) ;
B : contrôle négatif (inhibition maximale) : N^{G}-monométhyl-L-arginine (forme L) à 200 µM ;
C : contrôle de spécificité de l'inhibition : N^{G}-monométhyl-L-arginine (forme D) à 200 µM.

Pour déterminer l'activité du produit à tester on mesure la quantité de produits de réaction stables du NO (nitrites et nitrates) à l'aide du kit "nitric colorimetric assay" vendu par la société Boehringer sous la référence 1756.28.

L'extrait a été testé aux concentrations de 0,01%, 0,1 % et 0,25% (poids/volume)

| | | |
|---|---|---|
| Produit testé | | % inhibition |
| A | | 0 |
| B | | 100 |
| C | | 0 |
| 1 | 0,01% | 17 |
| 1 | 0,1% | 106 |
| 1 | 0,25% | 95 |
| 2 | 0,01% | 20 |
| 2 | 0,1% | 108 |
| 2 | 0,25% | 160 |

L'extrait présente un effet inhibiteur de la NO-synthase inductible.

### Exemple 2 :

Exemples de formulation illustrant l'invention. La composition a été obtenue par simple mélange des différents composants.

| Composition 1 : Gel | |
|---|---|
| *Olea europaea** | 1,00 % |
| Methylparaben | 0,20 % |
| Carbomer | 0,70 % |
| Polyethylène glycol (80E) | 10,00 % |
| Imidazolidinyl urée | 0,30 % |
| Triéthanolamine | 0,58 % |
| Eau | qsp 100 % |
| | |

| Composition 2 : Lotion | |
|---|---|
| *Olea europaea** | 2, 00 % |
| Antioxydant | 0,05 % |
| Isopropanol | 40,00 % |
| Conservateur | 0,30 % |
| Eau | qsp 100 % |
| | |

| Composition 3 : Gel pour le soin | |
|---|---|
| *Olea europaea** | 3,00 % |
| Hydroxypropylcellulose** | 1,00 % |
| Antioxydant | 0,05 % |
| Isopropanol | 40,00 % |
| Conservateur | 0,30 % |
| Eau | qsp 100 % |
| | |

| Composition 4 : Crème de soin (émulsion huile dans eau) | |
|---|---|
| *Olea europaea** | 5,00 % |
| Stéarate de glycérol | 2,00 % |
| Polysorbate 60***' | 1,00 % |
| Acide stéarique | 1,40 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100 % |
| | |

| Composition 5 : Shampooing | |
|---|---|
| *Olea europaea** | 0,50 % |
| Hydroxypropylcellulose** | 1,00 % |
| Parfum | 0,50 % |
| Conservateur | 0,30 % |
| Eau | qsp 100 % |
| | |

| Composition 6 : Crème de soin (émulsion huile/eau) | |
|---|---|
| *Olea europaea** | 5,00 %. |
| Stéarate de glycérol | 2,00 % |
| Polysorbate 60*** | 1,00 % |
| Acide stéarique | 1,40 % |
| Acide n-octanoyl-5-salicylique | 0,50 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau | qsp 100 % |
| | |

| Composition 7 : Gel anti-douleur | |
|---|---|
| *Olea europaea** | 5,00 % |
| Hydroxypropylcellulose** | 1,00 % |
| Antioxydant | 0,05 % |
| Chlorhydrate de lidocaïne | 2,00 % |
| Isopropanol | 40,00 % |
| Conservateur | 0,30 % |
| Eau | qsp 100 % |
| | |

| Composition 8 : Crème de soin de l'érythème solaire (émulsion huile-dans-eau) | |
|---|---|
| *Olea europaea** | 5,00 % |
| Stéarate de glycérol | 2,00 % |
| Polysorbate 60*** | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00% |
| Antioxydant | 0,05 % |
| Parfum | 0,50 % |
| Conservateur | 0,30 % |
| Eau | qsp 100 % |

| | |
|---|---|
| Composition 9 : Gel pour le traitement de l'acné | |
| *Olea europaea** | 5,00 % |
| Acide tout trans rétinoïque | 0,05 % |
| Hydroxypropylcellulose** | 1,00 % |
| Antioxydant | 0,05 % |
| Isopropanol | 40,00 % |
| Conservateur | 0,30 % |
| Eau | qsp 100 % |
| | |

| Composition 10 : Lotion pour éliminer les cicatrices dues à l'acné | |
|---|---|
| *Olea europaea** | 3,00 % |
| Acide glycolique | 50,00 % |
| Hydroxypropylcellulose** | 0,05 % |
| Conservateur | 0,30 % |
| NaOH | qsp pH = 2,8 |
| Ethanol | qsp 100 % |

| | |
|---|---|
| * : extrait aqueux sec de végétal de l'espèce *Olea europaea* vendu par la société Vinyals sous le nom commercial d'extrait sec d'olivier. **: Klucel H® vendu par la société Hercules ***: Tween 60® vendu par la société ICl | |

### Exemple 3 : Effet de la composition 1 de l'exemple 2 sur une peau sensible.

Il a été démontré que la peau sensible se caractérise par une réactivité neurosensorielle de la peau très forte à l'application topique sur la face de capsaïcine. (Magnusson et Koskinen, Acta Derm. Venereol.(Stockh), 1996, 76, 129-132).
La sensation de picotement est un des signes les plus prédictifs de la peau sensible
La composition de l'exemple 2 a donc été testée sur une population (15 personnes volontaires) de personnes présentant les caractéristiques d'une peau sensible, en comparaison de la même composition ne contenant pas d'extrait d' *Olea europaea.*

La composition et le contrôle sont appliqués sur la face à l'angle des joues, de manière aléatoire en double aveugle.
30 minutes après traitement, une crème à 0,075% de capsaïcine est appliquée sur les zones traitées.
La sensation de picotement est alors évaluée selon :
0 = aucun
1 = léger
2 = modéré
3 = fort
Les résultats moyens sont présentés dans le tableau suivant :

| Temps (mn) | Contrôle | Composition 1 |
|---|---|---|
| 5 | 1,00 | 0,87 |
| 10 | 1,53 | 1,33 |
| 15 | 1,80 | 1,53 |
| 20 | 1,60 | 1,47 |
| 25 | 1,07 | 0,87 |
| 30 | 0,60 | 0,47 |

Le gel contenant l'extrait d'*Olea europaea* présente un effet protecteur contre la sensation de picotement induit par la capsaïcine

## Revendications

1. Utilisation d'une quantité efficace d'au moins un extrait obtenu à partir de feuilles d'au moins un végétal de l'espèce *Olea europaea*, dans un milieu physiologiquement acceptable, pour préparer une composition destinée à ralentir voire inhiber la croissance de l'épiderme et/ou à traiter les désordres hyperprolifératifs de la peau.

2. Utilisation d'une quantité efficace d'au moins un extrait obtenu à partir de feuilles d'au moins un végétal de l'espèce *Olea europaea*, dans un milieu physiologiquement acceptable, pour préparer une composition destinée à traiter les processus inflammatoires neurogéniques cutanés.

3. Utilisation d'une quantité efficace d'au moins un extrait obtenu à partir de feuilles d'au moins un végétal de l'espèce *Olea europaea*, dans un milieu physiologiquement acceptable, pour préparer une composition destinée à traiter les peaux dites sensibles

4. Utilisation d'une quantité efficace d'au moins un extrait obtenu à partir de feuilles d'au moins un végétal de l'espèce *Olea europaea*, dans un milieu physiologiquement acceptable, pour préparer une composition destinée à traiter les érythèmes, particulièrement les érythèmes induits par les rayonnements ultra-violets.

5. Utilisation d'une quantité efficace d'au moins un extrait obtenu à partir de feuilles d'au moins un végétal de l'espèce *Olea europaea,* dans un milieu physiologiquement acceptable, pour préparer une composition destinée à traiter les éruptions érythémateuses localisées ou diffuses de la peau.

6. Utilisation d'une quantité efficace d'au moins un extrait obtenu à partir de feuilles d'au moins un végétal de l'espèce *Olea europaea*, dans un milieu physiologiquement acceptable, pour préparer une composition destinée à traiter la rosacée.

7. Utilisation d'une quantité efficace d'au moins un extrait obtenu à partir de feuilles d'au moins un végétal de l'espèce *Olea europaea*, dans un milieu physiologiquement acceptable, pour préparer une composition destinée à traiter les désordres de type hypermélanose.

8. Utilisation d'une quantité efficace d'au moins un extrait obtenu à partir de feuilles d'au moins un végétal de l'espèce *Olea europaea* selon l'une quelconque des revendications précédentes **caractérisée en ce que** la composition est destinée à inhiber la NO-synthase.

9. Utilisation cosmétique d'une quantité efficace d'au moins un extrait obtenu à partir de feuilles d'au moins un végétal de l'espèce *Olea Europaea,* dans une composition dans un milieu physiologiquement acceptable, ledit extrait étant destiné à traiter le vieillissement intrinsèque.

10. Utilisation cosmétique d'une quantité efficace d'au moins un extrait obtenu à partir de feuilles d'au moins un végétal de l'espèce *Olea Europaea,* dans une composition dans un milieu physiologiquement acceptable, ledit extrait étant destiné à inhiber la mélanogénèse induite par les rayonnements ultraviolets de type A et/ou B.

11. Utilisation cosmétique d'une quantité efficace d'au moins un extrait obtenu à partir de feuilles d'au moins un végétal de l'espèce *Olea Europaea,* dans une composition dans un milieu physiologiquement acceptable, ledit extrait étant destiné à contrôler la sudation.

12. Utilisation cosmétique d'une quantité efficace d'au moins un extrait obtenu à partir de feuilles d'au moins un végétal de l'espèce *Olea Europaea,* dans une composition dans un milieu physiologiquement acceptable, ledit extrait étant destiné à stimuler la lipolyse.

13. Utilisation cosmétique d'une quantité efficace d'au moins un extrait obtenu à partir de feuilles d'au moins un végétal de l'espèce *Olea Europaea,* dans une composition dans un milieu physiologiquement acceptable, ledit extrait étant destiné à renforcer fa fonction barrière de la peau.

14. Utilisation cosmétique d'une quantité efficace d'au moins un extrait obtenu à partir de feuilles d'au moins un végétal de l'espèce *Olea Europaea*, dans une composition dans un milieu physiologiquement acceptable, ledit extrait étant destiné à stimuler l'hydratation de la peau.

15. Utilisation cosmétique selon l'une quelconque des revendications 9 à 14, l'extrait étant destiné à inhiber la NO-synthase.

16. Utilisation selon l'une des revendications 9 à 15, ledit extrait étant utilisé dans une composition cosmétique, en application sur la peau, sur les cheveux, et/ou sur les muqueuses.

17. Utilisation selon l'une quelconque des revendications précédentes, **caractérisée par le fait que** l'extrait est en une quantité représentant de 10⁻⁴% à 20% du poids total de la composition.

18. Utilisation selon la revendication précédente, **caractérisée par le fait que** l'extrait est en une quantité représentant de 5.10⁻³% à 10% du poids total de la composition.

19. Utilisation selon l'une quelconque des revendications précédentes, **caractérisée par le fait que** l'extrait est obtenu à partir de matériel végétal issu d'au moins un végétal cultivé *in vivo*.

## Claims

1. Use of an effective amount of at least one extract obtained from leaves of at least one plant of the species *Olea europaea*, in a physiologically acceptable medium, for preparing a composition intended to slow down or even inhibit growth of the epidermis and/or to treat hyperproliferative disorders of the skin.

2. Use of an effective amount of at least one extract obtained from leaves of at least one plant of the species *Olea europaea*, in a physiologically acceptable medium, for preparing a composition intended to treat neurogenic inflammatory processes of the skin.

3. Use of an effective amount of at least one extract obtained from leaves of at least one plant of the species *Olea europaea*, in a physiologically acceptable medium, for preparing a composition intended to treat "sensitive" skin.

4. Use of an effective amount of at least one extract obtained from leaves of at least one plant of the species *Olea europaea*, in a physiologically acceptable medium, for preparing a composition intended to treat erythemas, particularly erythemas induced by ultraviolet radiation.

5. Use of an effective amount of at least one extract obtained from leaves of at least one plant of the species *Olea europaea,* in a physiologically acceptable medium, for preparing a composition intended to treat localized or diffuse erythematous eruptions of the skin.

6. Use of an effective amount of at least one extract obtained from leaves of at least one plant of the species *Olea europaea*, in a physiologically acceptable medium, for preparing a composition intended to treat rosacea.

7. Use of an effective amount of at least one extract obtained from leaves of at least one plant of the species *Olea europaea,* in a physiologically acceptable medium, for preparing a composition intended to treat disorders of the hypermelanosis type.

8. Use of an effective amount of at least one extract obtained from leaves of at least one plant of the species *Olea europaea*, according to any one of the preceding claims, **characterized in that** the composition is intended to inhibit NO-synthase.

9. Cosmetic use of an effective amount of at least one extract obtained from leaves of at least one plant of the species *Olea europaea*, in a composition in a physiologically acceptable medium, said extract being intended to treat intrinsic aging.

10. Cosmetic use of an effective amount of at least one extract obtained from leaves of at least one plant of the species *Olea europaea*, in a composition in a physiologically acceptable medium, said extract being intended to inhibit melanogenesis induced by ultraviolet radiation type A and/or B.

11. Cosmetic use of an effective amount of at least one extract obtained from leaves of at least one plant of the species *Olea europaea,* in a composition in a physiologically acceptable medium, said extract being intended to control sweating.

12. Cosmetic use of an effective amount of at least one extract obtained from leaves of at least one plant of the species *Olea europaea,* in a composition in a physiologically acceptable medium, said extract being intended to stimulate lipolysis.

13. Cosmetic use of an effective amount of at least one extract obtained from leaves of at least one plant of the species *Olea europaea,* in a composition in a physiologically acceptable medium, said extract being intended to reinforce the barrier function of the skin.

14. Cosmetic use of an effective amount of at least one extract obtained from leaves of at least one plant of the species *Olea europaea*, in a composition in a physiologically acceptable medium, said extract being intended to stimulate moisturization of the skin.

15. Cosmetic use according to any one of Claims 9 to 14, the extract being intended to inhibit NO-synthase.

16. Use according to one of Claims 9 to 15, said extract being used in a cosmetic composition, as an application to the skin, to the hair and/or to the mucous membranes.

17. Use according to any one of the preceding claims, **characterized in that** the extract is in an amount representing from 10⁻⁴% to 20% of the total weight of the composition.

18. Use according to the preceding claim, **characterized in that** the extract is in an amount representing from 5×10⁻³% to 10% of the total weight of the composition.

19. Use according to any one of the preceding claims, **characterized in that** the extract is obtained from plant material derived from at least one plant cultured *in vivo.*

## Patentansprüche

1. Verwendung einer wirksamen Menge mindestens eines Extraktes, der ausgehend von Blättern mindestens einer Pflanze der Art *Olea europaea* erhalten wurde, in einem physiologisch akzeptablen Medium, um eine Zusammensetzung herzustellen, die dazu vorgesehen ist, das Wachstum der Epidermis zu verlangsamen oder sogar zu inhibieren und/oder hyperproliferative Störungen der Haut zu behandeln.

2. Verwendung einer wirksamen Menge mindestens eines Extraktes, der ausgehend von Blättern mindestens einer Pflanze der Art *Olea europaea* erhalten wurde, in einem physiologisch akzeptablen Medium, um eine Zusammensetzung herzustellen, die dazu vorgesehen ist, entzündliche neurogene Prozesse der Haut zu behandeln.

3. Verwendung einer wirksamen Menge mindestens eines Extraktes, der ausgehend von Blättern mindestens einer Pflanze der Art *Olea europaea* erhalten wurde, in einem physiologisch akzeptablen Medium, um eine Zusammensetzung herzustellen, die dazu vorgesehen ist, so genannte empfindliche Hauttypen zu behandeln.

4. Verwendung einer wirksamen Menge mindestens eines Extraktes, der ausgehend von Blättern mindestens einer Pflanze der Art *Olea europaea* erhalten wurde, in einem physiologisch akzeptablen Medium, um eine Zusammensetzung herzustellen, die dazu vorgesehen ist, Erytheme zu behandeln, insbesondere durch UV-Stahlung hervorgerufene Erytheme.

5. Verwendung einer wirksamen Menge mindestens eines Extraktes, der ausgehend von Blättern mindestens einer Pflanze der Art *Olea europaea* erhalten wurde, in einem physiologisch akzeptablen Medium, um eine Zusammensetzung herzustellen, die dazu vorgesehen ist, lokale oder diffuse erythematöse Eruptionen zu behandeln.

6. Verwendung einer wirksamen Menge mindestens eines Extraktes, der ausgehend von Blättern mindestens einer Pflanze der Art *Olea europaea* erhalten wurde, in einem physiologisch akzeptablen Medium, um eine Zusammensetzung herzustellen, die dazu vorgesehen ist, Rosacea zu behandeln.

7. Verwendung einer wirksamen Menge mindestens eines Extraktes, der ausgehend von Blättern mindestens einer Pflanze der Art *Olea europaea* erhalten wurde, in einem physiologisch akzeptablen Medium, um eine Zusammensetzung herzustellen, die dazu vorgesehen ist, um Störungen vom Hypermelanosetyp zu behandeln.

8. Verwendung einer wirksamen Menge mindestens eines Extraktes, der aus Blättern mindestens einer Pflanze der Art *Olea europaea* erhalten wurde, nach einem der vorgehenden Ansprüche, **dadurch gekennzeichnet, dass** die Zusammensetzung dazu vorgesehen ist, die NO-Synthase zu inhibieren.

9. Kosmetische Verwendung einer wirksamen Menge mindestens eines Extraktes, der aus Blättern mindestens einer Pflanze der Art *Olea europaea* erhalten wurde, in einer Zusammensetzung in einem physiologisch akzeptablen Medium, wobei der Extrakt dazu vorgesehen ist, die intrinsische Alterung zu behandeln.

10. Kosmetische Verwendung einer wirksamen Menge mindestens eines Extraktes, der aus Blättern mindestens einer Pflanze der Art *Olea europaea* erhalten wurde, in einer Zusammensetzung in einem physiologisch akzeptablen Medium, wobei der Extrakt dazu vorgesehen ist, die durch UV-Strahlung vom Typ A und/oder B induzierte Melanogenese zu inhibieren.

11. Kosmetische Verwendung einer wirksamen Menge mindestens eines Extraktes, der aus Blättern mindestens einer Pflanze der Art *Olea europaea* erhalten wurde, in einer Zusammensetzung in einem physiologisch akzeptablen Medium, wobei der Extrakt dazu vorgesehen ist, die Schweißabsonderung zu kontrollieren.

12. Kosmetische Verwendung einer wirksamen Menge mindestens eines Extraktes, der aus Blättern mindestens einer Pflanze der Art *Olea europaea* erhalten wurde, in einer Zusammensetzung in einem physiologisch akzeptablen Medium, wobei der Extrakt dazu vorgesehen ist, die Lipolyse zu stimulieren.

13. Kosmetische Verwendung einer wirksamen Menge mindestens eines Extraktes, der aus Blättern mindestens einer Pflanze der Art *Olea europaea* erhalten wurde, in einer Zusammensetzung in einem physiologisch akzeptablen Medium, wobei der Extrakt dazu vorgesehen ist, die Barrierefunktion der Haut zu stärken.

14. Kosmetische Verwendung einer wirksamen Menge mindestens eines Extraktes, der aus Blättern mindestens einer Pflanze der Art *Olea europaea* erhalten wurde, in einer Zusammensetzung in einem physiologisch akzeptablen Medium, wobei der Extrakt dazu vorgesehen ist, die Hydratisierung der Haut zu stimulieren.

15. Kosmetische Verwendung nach einem der vorgehenden Ansprüche 9 bis 14, wobei der Extrakt dazu vorgesehen ist, die NO-Synthase zu inhibieren.

16. Verwendung nach einem der Ansprüche 9 bis 15, wobei der Extrakt in einer kosmetischen Zusammensetzung zur Anwendung auf die Haut, die Haare und/oder die Schleimhäute verwendet wird.

17. Verwendung nach einem der vorhergehenden Ansprüche, **dadurch gekennzeichnet, dass** der Extrakt in einer Menge von 10⁻⁴ bis 20 % des Gesamtgewichts der Zusammensetzung vorliegt.

18. Verwendung nach dem vorherigen Anspruch, **dadurch gekennzeichnet, dass** der Extrakt in einer Menge von 5·10⁻³ bis 10 % des Gesamtgewichts der Zusammensetzung vorliegt.

19. Verwendung nach einem der vorgehenden Ansprüche, **dadurch gekennzeichnet, dass** der Extrakt ausgehend von Pflanzenmaterial erhalten wird, das von mindestens einer *in vivo* kultivierten Pflanze stammt.
